(19) Europäisches Patentamt
European Patent Office
Office européen des brevets

(11) EP 2 718 229 B1

(12) **FASCICULE DE BREVET EUROPEEN**

(45) Date de publication et mention de la délivrance du brevet:
**25.03.2015 Bulletin 2015/13**

(21) Numéro de dépôt: **12731175.1**

(22) Date de dépôt: **07.06.2012**

(51) Int Cl.:
***C01B 31/02*** $^{(2006.01)}$ ***C07D 307/62*** $^{(2006.01)}$

(86) Numéro de dépôt international:
**PCT/IB2012/052884**

(87) Numéro de publication internationale:
**WO 2012/168899 (13.12.2012 Gazette 2012/50)**

(54) **PROCÉDÉ DE FONCTIONNALISATION SÉLECTIVE DE NANOTUBES DE CARBONE MONOPAROIS**

VERFAHREN ZUR SELEKTIVEN FUNKTIONALISIERUNG VON EINWANDIGEN KOHLENSTOFFNANORÖHREN

METHOD FOR SELECTIVE FUNCTIONALIZATION OF SINGLE-WALLED CARBON NANOTUBES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.06.2011 FR 1155137**

(43) Date de publication de la demande:
**16.04.2014 Bulletin 2014/16**

(73) Titulaire: **Commissariat à l'Énergie Atomique et aux Énergies Alternatives**
**75015 Paris (FR)**

(72) Inventeur: **CHENEVIER, Pascale**
**38700 La Tronche (FR)**

(74) Mandataire: **Majidi, Assieh et al**
**Cabinet Orès**
**36, rue de Saint-Pétersbourg**
**75008 Paris (FR)**

(56) Documents cités:
**WO-A2-2008/057070 WO-A2-2010/089395**
**JP-A- 2010 010 162**

- **HERSAM M C ET AL: "Sorting carbon nanotubes by electonic structure using density differentiation", NATURE NANOTECHNOLOGY, NATURE PUBLISHING GROUP, LONDON; GB, vol. 1, 4 octobre 2006 (2006-10-04), pages 60-66, XP002512670, DOI: 10.1038/NNANO.2006.52**
- **GRÉGORY SCHMIDT ET AL: "Labile Diazo Chemistry for Efficient Silencing of Metallic Carbon Nanotubes", CHEMISTRY - A EUROPEAN JOURNAL, vol. 17, no. 5, 1 février 2011 (2011-02-01), pages 1415-1418, XP055017996, ISSN: 0947-6539, DOI: 10.1002/chem.201002441 cité dans la demande**
- **DYKE C A ET AL: "Separation of single-walled carbon nanotubes on silica gel. Materials morphology and Raman excitation wavelength affect data interpretation", JOURNAL OF THE AMERICAN CHEMICAL SOCIETY, AMERICAN CHEMICAL SOCIETY, WASHINGTON, DC; US, vol. 127, no. 12, 30 mars 2005 (2005-03-30), pages 4497-4509, XP002391181, ISSN: 0002-7863, DOI: 10.1021/JA042828H cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 718 229 B1

## Description

La présente invention se rapporte à un procédé de fonctionnalisation sélective de nanotubes de carbone monoparois métalliques avec un dérivé de diazonium.

La présente invention se rapporte également à l'utilisation du mélange de nanotubes de carbone monoparois métalliques sélectivement fonctionnalisés et de nanotubes de carbone monoparois semi-conducteurs, obtenus par le procédé selon l'invention, pour la réalisation de canaux de transistors notamment en électronique, de matériaux accepteurs d'électrons notamment dans le photovoltaïque, d'émetteurs ou absorbeurs non linéaires de photons infra-rouge, d'électrodes conductrices de courant, d'électrodes transparentes souples, de revêtements antistatiques, de détecteurs chimiques, de cellules solaires.

L'invention a, en outre, pour objet un procédé de réalisation de canaux de transistors notamment en électronique, de matériaux accepteurs d'électrons notamment dans le photovoltaïque, d'émetteurs ou absorbeurs non linéaires de photons infra-rouge, d'électrodes conductrices de courant, d'électrodes transparentes souples, de revêtements antistatiques, de détecteurs chimiques, de cellules solaires, caractérisé en ce qu'il met en oeuvre le procédé de fonctionnalisation sélective de nanotubes de carbone monoparois métalliques selon l'invention.

Les nanotubes de carbone sont un matériau prometteur à la fois pour leurs qualités mécaniques, électroniques et optiques. Si des utilisations industrielles commencent à voir le jour dans le domaine du renfort mécanique par les nanotubes de carbone multiparois (MWNTs ou Multi-Wall Nanotubes en anglais), les applications en électronique et en optique sont plus lentes à venir. Dans ces domaines en effet, ce sont les nanotubes de carbone monoparois (SWNTs ou Single-Wall Nanotubes en anglais) qui montrent les propriétés les plus originales et les plus performantes.

Malheureusement, les nanotubes de carbone monoparois (SWNTs) peuvent être, selon la chiralité c'est-à-dire la géométrie de l'enroulement du plan carboné, soit métalliques (m-SWNTs) soit semi-conducteurs (sc-SWNTs). Quelle que soit la méthode de synthèse, les SWNTs métalliques et les SWNTs semi-conducteurs sont, généralement, synthétisés sous la forme d'un mélange des deux types.

Les SWNTs métalliques et semi-conducteurs sont tous deux intéressants mais pour des applications différentes. Lorsque les SWNTs métalliques et semi-conducteurs sont en mélange, la présence d'un type est souvent délétère pour l'utilisation de l'autre.

Par exemple, les SWNTs semi-conducteurs peuvent être utilisés comme canaux de transistors notamment en électronique ou comme matériaux accepteur d'électrons notamment dans le photovoltaïque ; la présence de SWNTs métalliques parmi les SWNT semi-conducteurs provoque alors des courts-circuits. Les SWNTs semi-conducteurs peuvent également être utilisés comme émetteurs non-linéaires de photons infra-rouge en photonique ; les SWNTs métalliques en contact avec les SWNTs semi-conducteurs provoquent l'extinction de la luminescence.

De la même façon, les SWNTs métalliques peuvent, par exemple, être utilisés comme matériaux pour des fils, vias ou électrodes conductrices de courant ; les SWNTs semi-conducteurs présents parmi les SWNTs métalliques provoquent une forte instabilité de la conductivité.

La séparation des SWNTs métalliques des SWNTs semi-conducteurs est donc un fort besoin identifié depuis leur découverte, et constitue le principal point bloquant pour leur utilisation industrielle. La séparation des SWNTs métalliques des SWNTs semi-conducteurs est ainsi devenue un sujet très concurrentiel et particulièrement actif depuis les années 2000.

Différentes méthodes de différenciation, d'enrichissement ou de séparation des SWNTs ont été déjà décrites dans la littérature.

L'une de ces méthodes consiste à effectuer un couplage sélectif des SWNTs métalliques avec le diazonium (Strano MS. et al., Science, 2003, 301, pp. 1519-1522) dans le but de séparer les SWNTs semi-conducteurs des SWNTs métalliques (Dyke CA. et al., Journal of the American Chemical Society, 2005, 127, p. 4497-4509). La sélectivité de cette réaction étant faible, elle ne permet ni d'obtenir une bonne séparation de la matière première ni une matière première de bonne qualité. En effet, la différenciation chimique sélective des SWNTs métalliques est difficile étant donné que la réactivité des SWNTs ne dépend pas seulement du type électronique des SWNTs mais aussi, en grande partie, de leur diamètre. Ainsi, les SWNTs semi-conducteurs ayant un petit diamètre (diamètre en général inférieur à 1,1 nm) ont donc tendance à réagir de la même manière que les SWNTs métalliques ayant un grand diamètre (diamètre en général supérieur à 1,1 nm) rendant ainsi leur séparation difficile.

D'autres équipes ont également utilisé la réaction des diazoniums sur les SWNTs pour différencier et séparer les SWNTs métalliques des SWNTs semi-conducteurs. Cependant, la sélectivité de la réaction est rarement donnée par les auteurs et il n'est pas toujours possible de la déterminer d'après les données publiées. Dans les réactions indiquées ci-dessous, la sélectivité a été estimée grossièrement d'après les tableaux et spectres publiés.

Le groupe de J. Tour est un pionnier de la réaction des diazoniums sur les SWNTs. Les travaux portent sur les SWNTs HiPco®, qui sont des SWNTs de petits diamètres c'est-à-dire un diamètre inférieur ou égal à 1,1 nm, en particulier un diamètre compris entre 0,9 et 1,1 nm, solubilisés dans des solutions aqueuses de SDS (laurylsulfate de sodium ou dodécylsulfate de sodium). D'après les tableaux et spectres publiés, la sélectivité vis-à-vis des SWNTs

métalliques peut être estimée à environ 7 (Doyle CD. Et al., Journal of the American Chemical Society 2008, 130, p. 6795-6800). Autrement dit, la vitesse de réaction des SWNTs métalliques est 7 fois plus grande que la vitesse de réaction des SWNTs semi-conducteurs.

Le groupe de M. Strano travaille sur cette réaction depuis 8 ans. Tous les travaux portent sur les SWNTs HiPco®, solubilisés dans une solution aqueuse de SDS, un tensioactif anionique, parfois comparé avec un tensioactif neutre comme le Triton. La sélectivité vis-à-vis des SWNTs métalliques peut être estimée à 6 environ dans les meilleurs cas, c'est-à-dire que la vitesse de réaction des SWNTs métalliques est d'environ 6 fois plus grande que la vitesse de réaction des SWNTs semi-conducteurs (Nair N. et al., Journal of the American Chemical Society, 2007, 129, p.3946-3954).

Wang et Shim ont publié une étude sur la fonctionnalisation sélective des SWNTs métalliques par les diazoniums par CVD (dépôt chimique en phase vapeur). La réactivité des SWNTs individuels est mesurée et comparée. La sélectivité peut être estimée autour de 5, c'est-à-dire que la vitesse de réaction des SWNTs métalliques est 5 fois plus grande que la vitesse de réaction des SWNTs semi-conducteurs (Wang CJ. et al., Journal of the American Chemical Society, 2005, 127, p.11460-11468).

Ghosh et Rao ont utilisé la réaction des diazoniums sur les SWNTs pour une séparation des SWNTs métalliques des SWNTs semi-conducteurs. Il s'agit de SWNTs obtenus par arc électrique (de grand diamètre, c'est-à-dire un diamètre supérieur à 1,1 nm, en particulier un diamètre compris entre 1,2 et 2 nm, bornes incluses) solubilisés dans le SDS. La sélectivité n'est pas accessible. Les SWNTs différenciés sont séparés par ultracentrifugation sur gradient de densité. Les nanotubes sont ensuite recuits (Ghosh S. et al., Nano Research, 2009, 2, p.183-191).

Lee et al. ont récemment utilisé la réaction des diazoniums sur les SWNTs pour supprimer la conductivité des SWNTs métalliques et utiliser le mélange non séparé comme source de SWNTs semi-conducteurs pour des applications en électronique. Il s'agit de SWNT CoMocat® (de petit diamètre, c'est-à-dire un diamètre compris entre 0,7 et 0,9 nm) dissous dans une solution aqueuse de SDS. La sélectivité vis-à-vis des SWNTs métalliques peut être estimée, d'après les spectres, à environ 3, c'est-à-dire que la vitesse de réaction des SWNTs métalliques est d'environ 3 fois plus grande que la vitesse de réaction des SWNTs semi-conducteurs (Lee CW. et al., Advanced Materials, 2010, 22, p. 1278).

Il est à noter que pour toutes ces méthodes, qu'elles soient basées sur le couplage covalent ou la complexation, la sélectivité et par conséquent la séparation, sont meilleures lorsqu'il s'agit de SWNTs de petits diamètres c'est-à-dire un diamètre inférieur ou égal à 1,1 nm (comme le CoMoCAT ou le HiPco). Dès lors qu'il s'agit de SWNTs de plus grands diamètres c'est-à-dire un diamètre supérieur à 1,1 nm, en particulier un diamètre compris entre 1,2 et 2 nm, bornes incluses, comme les SWNTs obtenus par arc électrique, par laser ou par CVD (dépôt chimique en phase vapeur), la sélectivité diminue. En effet, il a été constaté que les propriétés physiques et chimiques sont moins marquées lorsque le diamètre des SWNTs augmente, en particulier leur réactivité a tendance à diminuer. En raison de leur meilleur contact avec les électrodes métalliques, les SWNTs semi-conducteurs de plus grands diamètres sont préférés pour les dispositifs électroniques (Zhang L et al., Journal of the American Chemical Society, 2008, 130, p.2686-2691).

Par ailleurs, lors de la réaction des diazoniums, notamment les aryles diazoniums, avec les SWNTs, lesdits aryles diazoniums se montrent sélectifs envers les SWNTs métalliques mais pas spécifiques. Autrement dit, la réaction ne permet pas de fonctionnaliser uniquement les SWNTs métalliques et les diazoniums réagissent également avec les SWNTs semi-conducteurs avec une vitesse plus faible mais non négligeable.

La demande WO 2010089395 décrit un procédé de séparation de SWNTs reposant sur la réaction d'un dérivé de diazonium, le diazoester, sur les SWNTs pour différencier et séparer les SWNTs métalliques des SWNTs semi-conducteurs. Il en résulte une fonctionnalisation covalente des SWNTs métalliques environ 10 fois plus élevée que la fonctionnalisation des SWNTs semi-conducteurs. Le couplage supprimant la conductivité des SWNTs, les SWNTs métalliques ne peuvent plus produire de court-circuit. Le matériau peut ainsi être utilisé directement pour des applications en électronique comme récemment démontré (Schmidt G. et al., Chemistry European Journal, 2011, 17, p.1415-1418). Une fois différenciés, les SWNTs métalliques et les SWNTs semi-conducteurs sont séparés des uns des autres puis traités thermiquement à 400°C (Cabana J. et al., Journal of the American Chemical Society, 2007, 129, p. 2244), pour des applications plus exigeantes comme la photonique. Malgré une plus grande sélectivité de cette méthode par rapport aux méthodes déjà connues, la séparation des SWNTs de grands diamètres tels que définis précédemment, reste difficile.

Une méthode de complexation/d'adsorption a été récemment décrite, dans laquelle des SWNTs dissous dans une solution aqueuse de SDS (laurylsulfate de sodium ou dodécylsulfate de sodium) sont séparés par chromatographie sur gel d'agarose (Tanaka T et al., Nano Letters, 2009, 9, p. 1497-1500 et Moshammer K. et al ;, Nano Research, 2009, 599-606). Cependant, comme la plupart des méthodes de séparation, la séparation n'est pas parfaite et les SWNTs semi-conducteurs obtenus contiennent encore une faible fraction de SWNTs métalliques très conducteurs susceptibles de produire des court-circuits dans les dispositifs électroniques par exemple.

Parmi toutes les méthodes de différenciation, d'enrichissement ou de séparation des SWNTs décrites dans la littérature, à ce jour, seul le procédé de séparation par ultracentrifugation sur gradient de densité a abouti à la commercialisation de SWNTs semi-conducteurs et de SWNTs métalliques sous forme séparée (Arnold MS. et al., Nature Nanotechnology 2006, 1, p. 60-65). Les SWNTs semi-conducteurs et les SWNTs métalliques séparés selon ce procédé

sont commercialisés par la société Nanointegris au prix de 150$/mg pour une pureté de 90% et 800$/mg pour une pureté de 99%. Malgré le haut degré de pureté annoncé, les SWNTs semi-conducteurs purs à 99% ne sont pas faciles à utiliser. Par exemple, dans un canal de transistor utilisant de nombreux SWNTs en parallèle pour faire chuter la résistance globale (autour de 6kΩ par nanotube de carbone), un court-circuit dû à la présence d'un seul nanotube de carbone monoparoi métallique reste très probable.

Il subsiste donc un réel besoin d'un procédé permettant la différenciation des SWNTs métalliques par rapport aux SWNTs semi-conducteurs par une fonctionnalisation sélective desdits SWNTs métalliques palliant les inconvénients de l'art antérieur.

En particulier, il existe un réel besoin d'un procédé permettant la fonctionnalisation des SWNTs métalliques, une sélectivité significativement améliorée par rapport à celle décrite pour les procédés de l'art antérieur et ce indépendamment du diamètre des SWNTs.

Il existe, en outre, un réel besoin d'un procédé permettant la fonctionnalisation des SWNTs qui soit industriellement réalisable.

La présente invention a précisément pour but de répondre à ces besoins en fournissant un procédé de fonctionnalisation sélective de nanotubes de carbone monoparois métalliques, caractérisé en ce que l'on fait réagir, à pH < 5:

- une dispersion aqueuse comprenant un mélange de nanotubes de carbone monoparois métalliques et de nanotubes de carbone monoparois semi-conducteurs et un tensioactif cationique, avec
- un (Z)-diazoester ascorbique de formule (I) :

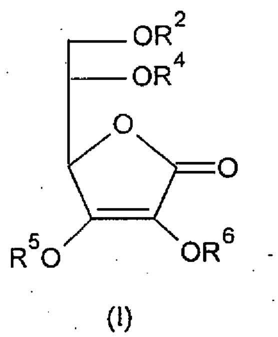

(I)

dans laquelle

- $R^5$ et $R^6$, différentes, sont choisis, indépendamment l'un de l'autre, parmi un atome d'hydrogène ou un groupe $R^1$-N=N- ;
- $R^1$ représente un groupe aryle en $C_{6-10}$ ou un groupe pyridyle, lesdits groupes aryle et pyridyle étant éventuellement substitués par un ou plusieurs substituants choisis parmi un atome d'halogène choisi parmi le chlore ou le brome,-$NO_2$, -$CO_2H$, -$CO_2R^3$, -$R^3$, ou -$OR^3$ ;
- $R^2$ et $R^4$, identiques ou différents, sont choisis, indépendamment l'un de l'autre, parmi un atome d'hydrogène, un groupe alkyle en $C_{1-20}$, -CO-alkyle en $C_{1-20}$, -$R^3$,-$CO_2R^3$, -CO-$NHR^3$ -CO-$NR^3R^7$ ;
- $R^3$ et $R^7$, identiques ou différents, sont choisis, indépendamment l'un de l'autre, parmi un groupe alkyle en $C_{1-20}$ ou un groupe halogène-alkyle en $C_{1-20}$.

Comme indiqué, $R^5$ est différent de $R^6$. Autrement dit, lorsque $R^5$ représente un atome d'hydrogène, $R^6$ représente un groupe $R^1$-N=N- et lorsque $R^5$ représente un groupe $R^1$-N=N-, $R^6$ représente un atome d'hydrogène.

Lorsque les trois conditions suivantes sont remplies, à savoir, l'utilisation d'un tensioactif cationique, le pH < 5 et l'utilisation d'un (Z)-diazoester ascorbique comme diazoester, une sélectivité de 20 à 100 entre la vitesse de couplage sur les SWNTs métalliques et sur les semi-conducteurs est obtenue, et ce aussi bien pour les SWNTs de petits diamètres (c'est-à-dire pour les diamètres inférieurs ou égaux à 1,1 nm) que les SWNTs de grands diamètres (c'est-à-dire pour les diamètres supérieurs à 1,1 nm).

Au sens de l'invention, par (Z)-diazoester ascorbique, on entend un composé de formule (I) dans lequel la double liaison -N=N- dans le groupe $R^1$-N=N- est de configuration Z.

Au sens de l'invention, par groupe « alkyle en $C_{1-20}$ » on entend, un groupe hydrocarboné monovalent linéaire, ramifié ou cyclique, saturé, éventuellement substitué, comprenant de 1 à 20 atomes de carbone. A titre indicatif, on peut citer les groupes méthyle, éthyle, propyle, butyle, isobutyle, pentyle, hexyle, octyle, nonyle, décyle, undécyle, dodécanyle, tridécyle, tétradécyle, pentadécyle, hexadécyle, heptadécyle, octadécyle, nonadécyle, eicosanyle et leurs isomères ramifiés. Comme alkyle cyclique, on peut également citer les groupes cyclopropyle, cyclobutyle, cyclopentyle, cyclohexyle, bicyclo[2,1,1] hexyle, bicycle[2,2,1] heptyle. Le groupe alkyle peut être éventuellement substitué par un ou

plusieurs hydroxyle, par un ou plusieurs groupe(s) aryle en $C_{6-10}$ tel que défini ci-dessous, par un ou plusieurs atomes d'halogène choisi parmi le fluor, le chlore, le brome ou l'iode. Le groupe alkyle peut, par exemple, comprendre de 1 à 16 atomes de carbone.

Le terme "halogène-alkyle en $C_{1-20}$", signifie une groupe alkyle en $C_{1-20}$ tel que défini ci-dessus comportant au moins un atome d'halogène choisi parmi le fluor, le chlore, le brome et/ou l'iode. De préférence, l'atome d'halogène est un atome de fluor. A titre d'exemple, on peut citer $-CF_3$, $-CH_2-CF_3$, $-C_2F_5$, $-CH(CF_3)_2$,$-CH(CH_3)(CF_3)$, $-C_3F_7$, $-C_4F_9$, $-C_5F_{11}$, $-C_6F_{13}$, $-C_7F_{15}$, $-C_8F_{17}$, $-C_9F_{19}$, $-C_{10}F_{21}$.

Le terme « aryle en $C_{6-10}$ » désigne de manière générale un substituant aromatique cyclique comportant de 6 à 10 atomes de carbone. Dans le cadre de l'invention, le groupe aryle peut être mono- ou poly- cyclique. A titre indicatif, on peut citer les groupes phényle et naphtyle. Le groupe aryle peut être éventuellement substitué par un ou plusieurs substituants choisis parmi un atome d'halogène choisi parmi le fluor, le chlore, le brome ou l'iode ; $-NO_2$; $-CO_2H$ ; $-CO_2R^3$, $-R^3$, ou $-OR^3$ avec $R^3$ étant un groupe alkyle en $C_{1-20}$ ou groupe halogène-alkyle en $C_{1-20}$, tel que défini précédemment.

Le terme « pyridyle », désigne un substituant aromatique hétérocyclique de formule $C_5H_5N$. Le groupe pyridyle peut être éventuellement substitué par un ou plusieurs substituants choisis parmi un atome d'halogène choisi parmi le fluor, le chlore, le brome ou l'iode ; $-NO_2$ ; $-CO_2H$ ; $-CO_2R^3$, $-R^3$, ou $-OR^3$ avec $R^3$ étant un groupe alkyle en $C_{1-20}$ ou groupe halogène-alkyle en $C_{1-20}$, tel que défini précédemment.

Par atome « d'halogène », on entend, dans le cadre de la présente invention, le fluor, le chlore, le brome ou l'iode.

Par « éventuellement substitué », on entend, un groupe dans lequel un, deux, trois ou plusieurs atomes peuvent être remplacés par un, deux, trois ou plusieurs substituants appropriés, y compris, sans limitation, un hydroxyle, un atome d'halogène, un groupe aryle en $C_{6-10}$ tel que défini ci-dessus, $-NO_2$; $-CO_2H$ ; $-CO_2R^3$, $-R^3$, ou $-OR^3$ avec $R^3$ étant un groupe alkyle en $C_{1-20}$ ou groupe halogène-alkyle en $C_{1-20}$, tel que défini précédemment.

Le terme « sélectivité » au sens de l'invention désigne le rapport des vitesses de réaction du réactif, en l'occurrence le (Z) diazoester ascorbique de formule (I), avec un type de nanotubes de carbone, en l'occurrence les SWNT métalliques, par rapport à l'autre type de nanotubes de carbone, en l'occurrence les SWNT semi-conducteurs. Une réaction sélective peut donc conduire à l'obtention d'un mélange de produits, l'un étant majoritaire et l'autre minoritaire.

Le terme « spécificité » au sens de l'invention désigne une réaction dans laquelle le réactif, en l'occurrence le (Z) diazoester ascorbique de formule (I), ne réagit qu'avec un seul type de nanotubes de carbone monoparois. Une réaction de sélectivité infinie pour un type de SWNTs est une réaction spécifique vis-à-vis de ce même type de SWNTs.

Dans le cadre de la présente invention, par « dispersion », on entend une suspension ou dispersion de deux phases distinctes : un milieu de dispersion (un solvant ou un mélange de solvants) et une phase dispersée (les nanotubes de carbone monoparois et le tensioactif cationique, par exemple). La dispersion ou suspension est dite stable, lorsque la phase dispersée (les nanotubes monoparois et le tensioactif cationique, par exemple) ne sédimentent pas. La dispersion est dite homogène, lorsque la phase dispersée dans le milieu de dispersion n'est pas visible à l'oeil nu ou au microscope optique. Lorsque la dispersion est homogène, elle peut également être considérée comme une « solution ». Ainsi, le terme « dispersion » englobe à la fois, les dispersions, les suspensions, et les solutions.

Lorsque l'un au moins des solvants du milieu de dispersion est l'eau, la dispersion ou la solution est dite aqueuse.

Au sens de l'invention, une solution tampon désigne un mélange constitué d'un acide faible (pour fournir des protons à un acide fort) et de sa base conjuguée (pour capter les protons d'un acide fort), ou d'une base faible (pour capter les protons d'un acide fort) et de son acide conjugué (pour transférer ses protons à une base forte) et qui permet de maintenir le pH d'un milieu constant malgré l'addition de petites quantités d'un acide ou d'une base, ou malgré une dilution.

Avantageusement, les nanotubes mis en oeuvre dans le cadre de la présente invention sont des nanotubes de carbone présentant :

- une longueur comprise entre 10 nm et 400 $\mu$m, en particulier entre 50 nm et 20 $\mu$m, plus particulièrement entre 200 nm et 2 $\mu$m, bornes incluses, et
- un diamètre compris entre 0,2 et 6 nm, en particulier entre 0,6 et 4 nm, plus particulièrement entre 0,7 et 2 nm, bornes incluses.

A titre d'exemple de nanotubes de carbone monoparois, on peut citer les SWNTs CoMocat®, Hipco®, les SWNTs obtenus par synthèse Laser (selon la synthèse décrite par O. Jost et al, Applied Physics Letters, 1999, 75, p. 2217), les SWNTs obtenus par arc électrique de source commerciale Nanoledge, Nanocarb® (Russie), Carbon Solutions (USA), ou encore les SWNTs obtenus par CVD (Nanocyl).

La concentration en nanotubes monoparois (SWNT) dans la dispersion aqueuse peut être comprise entre $1.10^{-5}$ et 10 g/l, avantageusement entre $1.10^{-4}$ et 1 g/l, et plus avantageusement entre $1.10^{-3}$ et $1.10^{-1}$ g/l, bornes incluses.

Le rapport SWNTs métalliques/SWNTs semi-conducteurs dans la dispersion aqueuse peut varier en fonction des conditions utilisées lors de la synthèse de ces nanotubes.

Préalablement à leur mise en dispersion et suite à leur préparation, les nanotubes de carbone peuvent subir

un traitement notamment pour retirer les particules métalliques de catalyseur utilisés lors de leur préparation et les sous-produits carbonés. Ainsi, toute technique connue de l'homme du métier permettant un tel traitement est utilisable dans le cadre de la présente invention.

A titre d'exemple de traitement, on peut citer un traitement du mélange des SWNTs métalliques et des SWNTs semi-conducteur par de l'acide nitrique et une chromatographie d'exclusion de taille dudit mélange suite audit traitement. Il est également possible d'appliquer un traitement ultrasonore avant, pendant et/ou après l'exposition à l'acide nitrique.

La mise en dispersion du mélange des nanotubes de carbone monoparois métalliques et des nanotubes de carbone semi-conducteurs peut se faire par tout moyen connu de l'homme du métier.

Comme indiqué précédemment, l'utilisation de tensioactifs avec les SWNTs est déjà décrite. Les tensioactifs classiquement utilisés sont les tensioactifs anioniques comme le SDS (le laurylsulfate de sodium ou dodécylsulfate de sodium), le SDBS (l'acide 4-dodécylbenzenesulfonique), le cholate de sodium, et les tensioactifs neutres comme le triton X100, les poloxamers. Dans le procédé de l'invention, le tensioactif est cationique.

Les tensioactifs cationiques de l'invention permettent non seulement de moduler la réactivité des SWNTs et du diazoester, mais en plus d'assurer une solubilisation optimale des SWNTs.

Dans le procédé de l'invention, les tensioactifs cationiques mis en oeuvre sont avantageusement choisis parmi le bromure de dodécyltriméthylammonium, le bromure d'hexadécyltriméthylammonium (dit CTAB), le bromure d'octadécyltriméthylammonium, le chlorure de dodécyltriméthylammonium, le chlorure d'hexadécylpyridinium, le poly(4-vinylpyridine-co-butylméthacrylate) ou le poly(4-vinylpyridine-co-styrène). De préférence, les tensioactifs cationiques sont choisis parmi le bromure de dodécyltriméthylammonium, le bromure d'hexadécyltriméthylammonium (dit CTAB), le bromure d'octadécyltriméthylammonium, le chlorure de dodécyltriméthylammonium ou le chlorure d'hexadécylpyridinium.

Le tensioactif cationique peut être introduit dans la dispersion comprenant les SWNTs tel quel ou en solution dans un solvant polaire, de préférence le même que celui de la dispersion des S WNTs.

Le tensioactif cationique dans la dispersion aqueuse est présent en une quantité comprise entre 0,001 et 10%, en particulier 0,01 et 5%, et plus particulièrement entre 0,2 et 1%, bornes incluses, en masse par rapport à la masse totale de la dispersion.

La dispersion comprenant le mélange des nanotubes et le tensioactif est aqueuse, ce qui signifie qu'elle contient au moins l'eau comme solvant.

Outre l'eau, ladite dispersion peut comprendre un ou plusieurs autres solvants polaires, de préférence protiques.

Par « solvant protique polaire », on entend un solvant qui présente une constante diélectrique, supérieure à 7, de préférence supérieure à 15, et qui possède un ou plusieurs hydrogènes acides. A titre indicatif, on peut citer l'eau, le méthanol, l'éthanol, le propanol, l'isopropanol, le n-butanol, l'acide formique.

La réaction des diazoniums de l'état de la technique avec les SWNTs utilise, en général, un milieu aqueux mais, le plus souvent, sans contrôle de pH.

Il a été constaté que lorsque dans le procédé de l'invention, le pH de la dispersion est maintenu à un pH acide, en particulier un pH < 5, la sélectivité et la spécificité de la fonctionnalisation des SWNTs métalliques par un (Z)-diazoester ascorbique de formule (I) est sensiblement améliorée.

Ainsi, le pH de la dispersion est plus particulièrement supérieur ou égal à 0 et inférieur à 5, de préférence entre 0,5 et 3,5, plus préférentiellement entre 1 et 2,5, bornes incluses.

Le pH de la dispersion peut être maintenu constant, par ajout d'une solution tampon. La solution tampon, lorsqu'elle est acide, peut être choisie parmi, par exemple, $CH_3CO_2H/CH_3CO_2^-$, $H_3PO_4/H_2PO_4^-$ ou $C_6H_5CO_2H/C_6H_5CO_2^-$. La solution tampon, lorsqu'elle est basique, peut être choisie parmi, par exemple, $NH_4^+/NH_3$, $(CH_3)_3NH^+/(CH_3)_3N$, $H_2PO_4^-/HPO_4^{2-}$. De préférence, le pH de la dispersion est maintenu constant par ajout d'une solution tampon, acide. L'homme du métier saura déterminer la quantité de solution tampon à mettre en oeuvre pour maintenir le pH de la dispersion aux valeurs de pH souhaitées.

Dans le procédé de l'invention, le (Z)-diazoester ascorbique de formule (I) peut être obtenu par réaction d'un sel de diazonium de formule (II) avec un composé de formule (III).

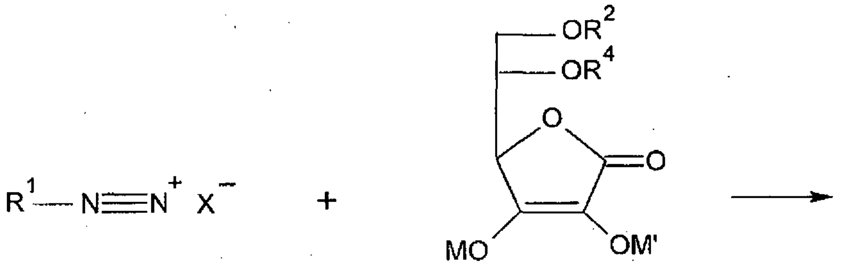

(II) (III) (I)

dans lesquelles

- $R^1$, $R^2$, et $R^4$ sont tels que définis précédemment ;
- M et M', identiques ou différents, sont choisis, indépendamment l'un de l'autre, parmi un atome d'hydrogène, un métal alcalin choisi parmi le sodium ou le potassium ;
- $X^-$ représente un atome d'halogène choisi parmi le fluor, le chlore, le brome ou l'iode ; ou un anion choisi parmi $BF_4^-$, $ClO_4^-$, ou $SO_3^-$.

Selon une variante de l'invention, M est différent de M'. Autrement dit, lorsque M représente un atome d'hydrogène, M' représente un métal alcalin choisi parmi le sodium ou le potassium et lorsque M représente un métal alcalin choisi parmi le sodium ou le potassium, M' représente un atome d'hydrogène.

Les sels de diazoniums sont connus depuis longtemps pour former des esters (appelés dans certains cas éthers) avec les composés oxygénés, d'abord sous la forme de (Z)-diazoesters, qui se transforment rapidement en (E)-diazoesters par un changement de conformation. Le diazoester ascorbique de conformation (E) est remarquablement stable et est inactif vis-à-vis du couplage sur les SWNTs (Zollinger H, VCH, 1994, p. 108-115 ; Doyle MP. et al., Journal of Organic Chemistry, 1989, 54, p. 3785-3789 ; G. Schmidt et al., Chemistry - a European Journal, 2011, 17, p.1415).

Le procédé de l'invention permet donc la réaction des SWNTs avec un diazoester de conformation (Z) dont la réactivité a été adaptée pour permettre une sélectivité bien plus grande que celle décrite avec les diazoniums ou les diazoesters de l'état de la technique.

De préférence, les sels de diazonium de formule (II) sont choisis dans le groupe constitué par le tétrafluoroborate de 4-nitrobenzène diazonium, le tétrafluoroborate 4-diazobenzoate de méthyle, le tétrafluoroborate d'acide 4-diazobenzoïque, le tétrafluoroborate d'acide 4-diazophthalique, le tétrafluoroborate de 4-diazophtalate de diméthyle.

A titre de composés de formule (III), il est particulièrement avantageux d'utiliser un composé choisi dans le groupe constitué par l'acide ascorbique, l'ascorbate de sodium ou le 6-O-palmitoylascorbate de sodium.

Le (Z)-diazoester ascorbique de formule (I) peut être préparé séparément, avant son introduction dans la dispersion aqueuse comprenant les SWNTs, par un procédé mettant en oeuvre les étapes consistant à :

a) préparer ou prendre une solution aqueuse du composé de formule (III) ;
b) introduire le sel de diazonium de formule (II) tel que défini précédemment dans la solution obtenue à l'étape a),
c) introduire la solution issue de l'étape b) dans la dispersion aqueuse comprenant les SWNTs et le tensioactif cationique.

Il est à noter que, lors de l'introduction la solution issue de l'étape b) dans la dispersion aqueuse de SWNTS, le pH de ladite dispersion aqueuse est maintenu à un pH < 5.

Comme indiqué, la solution de l'étape a) est une solution aqueuse signifiant que le solvant est au moins de l'eau. L'eau peut éventuellement être en mélange avec un ou plusieurs solvant(s) « protique(s) polaire(s) » tel(s) que défini(s) précédemment. De préférence, le(s) solvant(s) est (sont) le(s) même(s) que celui (ceux) de la dispersion. Le solvant de l'étape a) est avantageusement de l'eau.

La solution aqueuse de l'étape a) peut être préparée en mélangeant le composé de formule (III) avec le(s) solvant(s) sous une agitation manuelle.

La durée de l'étape b) peut aller de 1 seconde à 1 heure, par exemple de 3 secondes à 10 minutes et en particulier de 5 secondes à 1 minute, bornes incluses.

La solution issue de l'étape b) est, de préférence, introduite immédiatement dans ladite dispersion. Par « immédiatement », on entend dès que le sel de diazonium de formule (II) forme une solution homogène dans laquelle ledit sel est complètement dissous ou, tout au moins, paraît comme étant complètement dissous à l'oeil nu ou au

microscope optique.

Alternativement, le (Z)-diazoester ascorbique de formule (I) peut être préparé *in situ* par un procédé mettant en oeuvre les étapes consistant à :

i) introduire une solution aqueuse du composé de formule (III) dans la dispersion comprenant les SWNTs et le tensioactif cationique ; et
ii) introduire une solution aqueuse du sel de diazonium dans la dispersion issue de l'étape i).

Au cours de ce procédé, le pH de ladite dispersion est maintenu à pH < 5. Dans cette variante de procédé, les conditions opératoires sont les mêmes que celle de la variante précédente notamment en termes de moyens d'agitation, de durée de réaction et le choix de solvant(s).

Que le (Z)-diazoester de formule (I) soit préparé séparément ou *in situ,* le rapport molaire entre le sel de diazonium de formule (II) et le composé de formule (III) est de 0,001 à 1,5, de préférence de 0,01 à 1 équivalent molaire, bornes incluses.

Que le (Z)-diazoester de formule (I) soit préparé séparément ou *in situ,* la mise en contact et donc la réaction entre le sel de diazonium de formule (II) et le composé de formule (III) est effectuée à une température comprise entre 0 et 50°C, par exemple entre 1 et 40°C, bornes incluses, avantageusement, à la température du réfrigérateur, c'est-à-dire à une température de 4°C ± 3°C.

Selon une variante préférée de l'invention, le (Z)-diazoester de formule (I) est préparé séparément. Ainsi, l'étape b), c'est-à-dire l'étape au cours de laquelle les composés de formules (II) et (III) sont mélangés, est réalisée à 4°C± 3°C afin de limiter au maximum l'isomérisation spontanée du (Z)-diazoester en (E)-diazoester ascorbique. Toutefois, l'étape c), c'est-à-dire l'étape au cours de laquelle la solution issue de l'étape b) est ajoutée à la dispersion aqueuse de SWNTs, est réalisée à température ambiante (20°C ± 5°C).

Comme indiqué, le (Z)-diazoester ascorbique se transforme spontanément en (E)-diazoester ascorbique par une réaction d'isomérisation (Z)-(E) assez rapide. Comme le (E)-diazoester n'est pas réactif vis-à-vis des SWNTs, il est essentiel de maîtriser cette réaction d'isomérisation par un choix astucieux de conditions réactionnelles.

Dans le procédé de l'invention, la fonctionnalisation des SWNTs est effectuée avec le (Z)-diazoester de formule (I) dont la concentration dans la dispersion du mélange de nanotubes est de $1.10^{-9}$ à 1 mol/l, en particulier de $1.10^{-7}$ à $1.10^{-2}$ mol/l, plus particulièrement encore de $1.10^{-6}$ à $1.10^{-4}$ mol/l.

Dans le procédé de l'invention, la réaction du (Z)-diazoester ascorbique de formule (I) avec la dispersion de SWNTs, a lieu entre 0 et 50°C, de préférence entre 20 et 40°C, bornes incluses. Plus préférentiellement, la réaction du (Z)-diazoester ascorbique de formule (I) avec la dispersion de SWNTs a lieu à température ambiante, c'est-à-dire à une température de 20°C ± 5°C.

La durée de réaction entre la dispersion comprenant les SWNTs et le (Z)-diazoester ascorbique de formule (I) est comprise entre 5 minutes et 48 heures, en particulier entre 15 minutes et 3 heures, plus particulièrement entre 15 minutes et 1 heure, bornes incluses.

Lors de la préparation des différentes dispersions et/ou solutions mises en oeuvre dans le cadre de la présente invention, celles-ci sont soumises à une agitation. A titre d'exemple de moyen d'agitation, on peut citer une agitation manuelle, un traitement aux ultrasons, une agitation mécanique ou une combinaison de telles techniques. Ces techniques peuvent nécessiter l'utilisation d'un agitateur magnétique, d'un barreau aimanté, d'un bain à ultrasons, d'un agitateur mécanique à tiges, pales, hélices, etc. L'homme du métier saura choisir le moyen d'agitation adapté à chaque cas.

Il est possible d'utiliser le mélange obtenu par le procédé de l'invention tel quel. En effet, suite à la réaction du (Z)-diazoester ascorbique de formule (I) avec le mélange des SWNTs métalliques et des SWNTs semi-conducteurs, les propriétés des SWNTs métalliques sont très fortement modifiées par la réaction, alors que les SWNTs semi-conducteurs sont préservés grâce au caractère très sélectif du procédé de l'invention (la conjonction de l'utilisation du (Z)-diazoester ascorbique de formule (I), du tensioactif cationique et du pH < 5. Aussi, le mélange des SWNTs métalliques ainsi fonctionnalisés et de SWNTs semi-conducteurs peut être considéré comme une source de SWNTs semi-conducteurs « purs ». Un tel mélange peut, de ce fait être utilisé pour la réalisation de canaux de transistors notamment en électronique, de matériaux accepteurs d'électrons notamment dans le photovoltaïque, d'émetteurs ou d'absorbeurs non linéaires de photons infra-rouge, d'électrodes conductrices de courant, d'électrodes transparentes souples, de revêtements antistatiques, de détecteurs chimiques, et de cellules solaires.

Le procédé selon l'invention peut être suivi d'une étape de séparation des nanotubes de carbones monoparois métalliques fonctionnalisés des nanotubes de carbone monoparois semi-conducteurs.

La séparation des SWNTs métalliques fonctionnalisés des SWNTs semi-conducteurs peut se faire par toute technique de séparation connue dans ce domaine, comme par exemple, une technique de séparation basée sur l'affinité chimique, sur la filtration, sur la centrifugation, sur l'électrophorèse et/ou sur la chromatographie.

Le procédé décrit dans la demande de brevet japonais JP 2007 031238 peut également être utilisé pour effectuer une telle séparation.

Outre l'étape de séparation, le procédé de l'invention peut également comprendre une étape de traitement thermique des nanotubes séparés.

Ainsi, après leur séparation, les SWNTs métalliques fonctionnalisés et/ou les SWNTs semi-conducteurs peuvent subir une étape de traitement thermique. Le traitement thermique peut être effectué à sec comme cela est décrit par Cabana J. et al., Journal of the American Chemical Society, 2007, 129, p.2244 ou par Ghosh S. et al., Nano Research 2009, 2, p.183-191. Le traitement thermique peut également être effectué en solution comme décrit par Dyke C.A. et al., Synlett, 2004, 1, p. 155-160. La présente invention a pour objet un mélange de nanotubes de carbone monoparois métalliques sélectivement fonctionnalisés et de nanotubes de carbone monoparois semi-conducteurs, obtenu par le procédé de l'invention.

Un autre objet de la présente invention est l'utilisation d'un mélange de nanotubes de carbone monoparois métalliques sélectivement fonctionnalisés et de nanotubes de carbone monoparois semi-conducteurs, obtenu par le procédé de l'invention, pour la réalisation de canaux de transistors notamment en électronique, de matériaux accepteurs d'électrons notamment dans le photovoltaïque, d'émetteurs ou absorbeurs non linéaires de photons infra-rouge, d'électrodes conductrices de courant, d'électrodes transparentes souples, de revêtements antistatiques, de détecteurs chimiques, et de cellules solaires.

La présente invention concerne également les nanotubes de carbone monoparois métalliques fonctionnalisés obtenus par la séparation des nanotubes de carbone monoparois métalliques fonctionnalisés des nanotubes de carbone monoparois semi-conducteurs qui sont dans le mélange obtenu par le procédé de l'invention, ainsi que les utilisations desdits nanotubes de carbone monoparois métalliques fonctionnalisés séparés pour la réalisation d'électrodes conductrices de courant, d'électrodes transparentes souples, de revêtements antistatiques, de vias et interconnections en électronique, de câbles de conduction du courant, et de cellules solaires.

L'invention concerne encore les nanotubes de carbone monoparois métalliques fonctionnalisés séparés comme indiqué ci-dessus et ayant subi un traitement thermique, pour la réalisation d'électrodes conductrices de courant, d'électrodes transparentes souples, de revêtements antistatiques, de vias et interconnections en électronique, de câbles de conduction du courant, et de cellules solaires.

Le traitement thermique des nanotubes de carbone monoparois métalliques fonctionnalisés conduit à leur défonctionnalisation. Ce traitement peut intervenir, par exemple, à la fin de l'assemblage du système qui utilise les nanotubes de carbone monoparois métalliques fonctionnalisés, par exemple pour faciliter leur positionnement ou les protéger d'un endommagement dans une étape de procédé.

L'invention se rapporte également aux nanotubes de carbone monoparois semi-conducteurs obtenus lors de l'étape de séparation des nanotubes de carbone monoparois métalliques fonctionnalisés des nanotubes de carbone monoparois semi-conducteurs, ainsi que les utilisations desdits nanotubes de carbone monoparois semiconducteurs séparés pour la réalisation de canaux de transistors notamment en électronique, de matériaux accepteurs d'électrons notamment dans le photovoltaïque, d'émetteurs ou absorbeurs non linéaires de photons infra-rouge, d'électrodes conductrices de courant, d'électrodes transparentes souples, de revêtements antistatiques, de détecteurs chimiques, et de cellules solaires.

L'invention se rapporte, en outre, aux nanotubes de carbone monoparois semi-conducteurs séparés comme indiqué ci-dessus, ayant subi un traitement thermique, pour la réalisation d'émetteurs ou absorbeurs non linéaires de photons infra-rouge.

L'invention a, en outre, pour objet un procédé de réalisation de canaux de transistors notamment en électronique, de matériaux accepteurs d'électrons notamment dans le photovoltaïque, d'émetteurs ou absorbeurs non linéaires de photons infra-rouge, d'électrodes conductrices de courant, d'électrodes transparentes souples, de revêtements antistatiques, de détecteurs chimiques, de vias et interconnections en électronique, de câbles de conduction du courant, et de cellules solaires, caractérisé en ce qu'il met en oeuvre le procédé de fonctionnalisation sélective de nanotubes de carbone monoparois métalliques selon l'invention.

La présente invention concerne également un procédé de séparation de nanotubes de carbone monoparois métalliques des nanotubes de carbone monoparois semi-conducteurs comprenant une étape de fonctionnalisation sélective des nanotubes de carbone monoparois métalliques selon le procédé de l'invention.

D'autres avantages et caractéristiques de la présente invention pourront encore apparaître à la lecture des exemples ci-dessous donnés à titre illustratif et les figures annexées :

- la Figure 1 représente un exemple de fonctionnalisation sélective des SWNTs métalliques dans un mélange de SWNTs métalliques et de SWNTs semi-conducteurs CoMocat® et dans un mélange de SWNTs métalliques et de SWNTs semi-conducteurs HiPco®. La figure montre les spectres d'absorbance corrigée en fonction de la longueur d'onde en nm, de SWNTs en solution aqueuse dans le CTAB à pH 2,1 avant et après réaction avec le (Z)-diazoester issu de la réaction de l'acide ascorbique avec l'acide 4-diazophthalique. Les spectres d'absorbance du mélange des SWNTs avant fonctionnalisation (pointillés) et après fonctionnalisation (trait plein) sont corrigés selon la formule

absorbance corrigée = (absorbance – A x 1240 / (longueur d'onde en nm)) / A,

où A est une constante choisie au mieux pour chaque type de SWNT pour mieux montrer les pics d'absorption sur une courbe à plat, selon une procédure bien connue. Ainsi, pour obtenir l'absorbance corrigée, l'absorbance due au bruit de fond est soustraite de l'absorbance totale. La fonctionnalisation des SWNTs métalliques avec le (Z)-diazoester provoque la suppression des pics d'absorption correspondants entre 450 et 530nm pour les SWNTs CoMocat®, entre 500 et 600 nm pour les SWNTs HiPco®, alors que les pics des SWNTs semi-conducteurs à plus grande longueur d'onde ne sont pas ou très peu affectés ;

- la Figure 2 représente un exemple de fonctionnalisation sélective des SWNTs métalliques dans un mélange de SWNTs métalliques et de SWNTs semi-conducteurs obtenus par Laser (selon O. Jost et al, Applied Physics Letters, 1999, 75, p. 2217) et dans un mélange de SWNTs métalliques et de SWNTs semi-conducteurs obtenus par arc électrique Nanoledge. La figure montre les spectres d'absorbance corrigée en fonction de la longueur d'onde en nm, de SWNTs en solution aqueuse dans le CTAB à pH 2,1 avant et après réaction avec le (Z)-diazoester issu de la réaction de l'acide ascorbique avec l'acide 4-diazophthalique. Les spectres d'absorption du mélange des SWNTs avant fonctionnalisation (pointillés) et après fonctionnalisation (trait plein) sont corrigés selon la formule d'absorbance corrigée décrite pour la Figure 1. Dans les deux types de mélange de SWNTs Laser et Nanoledge, la fonctionnalisation des SWNTs métalliques avec le (Z)-diazoester provoque la suppression des pics d'absorption correspondants entre 600 et 800 nm, alors que les pics des SWNTs semi-conducteurs à plus grande longueur d'onde ne sont pas ou très peu affectés ;
- la Figure 3 représente un exemple de fonctionnalisation sélective des SWNTs métalliques dans un mélange de SWNTs métalliques et de SWNTs semi-conducteurs obtenus par arc électrique NanoCarb®, et dans un mélange de SWNTs métalliques et de SWNTs semi-conducteurs obtenus par arc électrique Carbon Solutions. La figure montre les spectres d'absorbance corrigée en fonction de la longueur d'onde en nm, de SWNTs en solution aqueuse dans le CTAB à pH 2,1 avant et après réaction avec le (Z)-diazoester issu de la réaction de l'acide ascorbique avec l'acide 4-diazophthalique. Les spectres d'absorption des SWNTs avant fonctionnalisation (pointillés) et après fonctionnalisation (trait plein) sont corrigés selon la formule d'absorbance corrigée décrite pour la Figure 1. Dans les deux types de mélange de SWNTs NanoCarb® et Carbon Solutions, la fonctionnalisation des SWNTs métalliques avec le (Z)-diazoester provoque la suppression des pics d'absorption correspondants entre 600 et 800 nm, alors que les pics des SWNTs semi-conducteurs à plus grande longueur d'onde ne sont pas ou très peu affectés ;
- la Figure 4 représente les spectres d'absorbance corrigée d'un mélange de SWNTs métalliques et de SWNTs semi-conducteurs de type Carbon Solutions en fonction de la longueur d'onde. Pour une meilleure comparaison, les spectres sont renormalisés selon la formule

absorbance corrigée = absorbance – A x 1240 / (longueur d'onde en nm)) / A

où A est l'absorbance à 1240 nm. Les pics d'absorption des SWNTs semi-conducteurs apparaissent entre 900 et 1200nm et entre 400 et 600 nm, alors que les pics d'absorption des SWNTs métalliques sont visibles entre 600 et 800nm. La courbe en trait fin gris montre le spectre de la solution du mélange de SWNTs après fonctionnalisation et avant séparation. La courbe en trait pointillé montre le spectre du surnageant après centrifugation, avec un pic de SWNTs métalliques considérablement réduit et des pics de SWNTs semi-conducteurs légèrement augmentés. La courbe en trait épais noir montre le spectre du culot de centrifugation remis en suspension dans l'eau, avec, au contraire, un pic de SWNTs métalliques augmenté et des pics de SWNTs semi-conducteurs légèrement réduits.

## EXEMPLES

### EXEMPLE :

Préparation de la dispersion du mélange de nanotubes de carbone monoparois métalliques et de nanotubes de carbone monoparois semi-conducteurs

Le mélange de SWNTs métalliques et de SWNTs semi-conducteurs est pesé et mélangé dans un flacon en verre avec une solution de CTAB, typiquement 20mg de SWNTs dans 20mL de solution de CTAB à 0,2% en masse par rapport à la masse totale de la dispersion.

L'ensemble est passé aux ultrasons pour former une dispersion homogène. Typiquement, on utilise une sonde à ultrasons trempée dans la solution en puissance 15W pendant 15minutes. La dispersion/solution est ensuite centrifugée

pour retirer les résidus de catalyseurs métalliques, les particules de carbone amorphe et les nanotubes de carbone mal dispersés, typiquement à 100000g pendant 30 minutes à 25°C. Le surnageant est prélevé et utilisé comme solution de SWNTs.

Fonctionnalisation des nanotubes de carbone monoparois métalliques par couplage avec le (Z)-diazoester ascorbique

La dispersion/solution du mélange de SWNTs obtenue précédemment est tamponnée à pH 2,1 par ajout d'un tampon phosphate. Typiquement on ajoute 100μL de solution de $H_3PO_4$ 0,5M + $KH_2PO_4$ 0,5M à 1mL de solution de SWNTs.

On prépare extemporanément une solution d'acide ascorbique à 10mM dans l'eau. On pèse quelques milligrammes de sel de diazonium, typiquement le tétrafluoroborate de 4-nitrobenzène diazonium ou le tétrafluoroborate d'acide 4-diazophthalique. Le sel de diazonium est dissout à 10mM dans la solution d'acide ascorbique et le mélange est immédiatement ajouté sous agitation à la solution de SWNTs en proportion typiquement de 10μL de ce mélange pour 1mL de solution de SWNTs tamponnée.

Alternativement, l'acide ascorbique peut être ajouté directement à la solution de SWNTs, typiquement en proportion 10μL d'acide ascorbique à 10mM dans l'eau pour 1mL de solution de SWNTs. Le diazonium est dissout à 1mM dans l'eau. Cette solution est ensuite ajoutée à la solution de SWNTs typiquement en une proportion de 100μL de solution de diazonium à 1mM pour 1mL de solution de SWNTs.

Les points critiques sont la bonne dispersion/solubilisation du mélange de SWNTs, l'utilisation d'un tensioactif cationique, le pH acide, le mélange équimolaire d'acide ascorbique et de sel de diazonium, et le contact rapide du mélange avec la dispersion/solution de SWNTs.

Les conditions d'ultracentrifugation sont soit une ultrasonication en bain à ultrasons de puissance 80W 45kHz à pleine puissance pendant 30 minutes à 2 heures, soit une ultrasonication avec une sonde à ultrasons trempée dans la solution en puissance 15W pendant 15 minutes à 2 heures.

Les conditions de centrifugation sont : une durée de 30 minutes à une température de 10 à 25°C avec une accélération de 80000 à 120000g.

Il a été constaté que lorsque les trois conditions suivante sont remplies, à savoir, l'utilisation d'un tensioactif cationique, un pH acide et l'utilisation du (Z)-diazoester ascorbique de formule (I), la fonctionnalisation des SWNTs métalliques est hautement sélective (les SWNTs semiconducteurs sont très peu ou pas du tout affectés par la réaction de couplage), et ce aussi bien pour les SWNTs de petits diamètres HiPco et CoMocat (c'est-à-dire pour les diamètres inférieurs à 1 nm) que pour les SWNTs de grands diamètres obtenus par laser et par arc électrique (Nanoledge, NanoCarb, Carbon Solutions, c'est-à-dire pour les diamètres supérieurs à 1 nm).

Séparation des nanotubes de carbone monoparois métalliques fonctionnalisés des nanotubes de carbone monoparois semiconducteurs non fonctionnalisés

Après la fonctionnalisation nanotubes de carbone monoparois métalliques par l'ester ascorbique de l'acide 4-diazophthalique, le milieu réactionnel est centrifugé à 4°C à une vitesse de 60000 à 100000g pendant 45 minutes. Le surnageant prélevé est enrichi en SWNTs semi-conducteurs et appauvri en SWNTs métalliques fonctionnalisés.

La Figure 4 montre les spectres d'absorbance corrigée de SWNTs de type Carbon Solutions en fonction de la longueur d'onde. Les pics d'absorption des SWNTs semi-conducteurs apparaissent entre 900 et 1200 nm et entre 400 et 600 nm, alors que les pics d'absorption des SWNTs métalliques sont visibles entre 600 et 800 nm. La courbe en trait fin gris montre le spectre de la solution de SWNTs après différentiation et avant séparation. La courbe en trait pointillé montre le spectre du surnageant après centrifugation, avec un pic de SWNTs métalliques considérablement réduit et des pics de SWNTs semi-conducteurs légèrement augmentés. La courbe en trait épais noir montre le spectre du culot de centrifugation remis en suspension dans l'eau, avec, au contraire, un pic de SWNTs métalliques augmenté et des pics de SWNTs semi-conducteurs légèrement réduits.

## Revendications

1. Procédé de fonctionnalisation sélective de nanotubes de carbone monoparois métalliques, **caractérisé en ce que** l'on fait réagir, à pH < 5 :

   - une dispersion aqueuse comprenant un mélange de nanotubes de carbone monoparois métalliques et de nanotubes de carbone monoparois semi-conducteurs et un tensioactif cationique, avec
   - un (Z)-diazoester ascorbique de formule (I) :

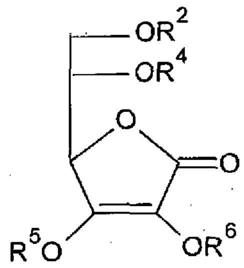

(I)

dans laquelle

- $R^5$ et $R^6$, différents, sont choisis, indépendamment l'un de l'autre, parmi un atome d'hydrogène ou un groupe $R^1$-N=N- ;
- $R^1$ représente un groupe aryle en $C_{6-10}$ ou un groupe pyridyle, lesdits groupes aryle et pyridyle étant éventuellement substitués par un ou plusieurs substituants choisis parmi un atome d'halogène choisi parmi le chlore ou le brome, $-NO_2$, $-CO_2H$, $-CO_2R^3$, $-R^3$ ou $-OR^3$ ;
- $R^2$ et $R^4$, identiques ou différents, sont choisis, indépendamment l'un de l'autre, parmi un atome d'hydrogène, un groupe alkyle en $C_{1-20}$, -CO-alkyle en $C_{1-20}$, $-R^3$,$-CO_2R^3$, $-CO-NHR^3$, $-CO-NR^3R^7$,
- $R^3$ et $R^7$, identiques ou différents, sont choisis, indépendamment l'un de l'autre, parmi un groupe alkyle en $C_{1-20}$ ou un groupe halogène-alkyle en $C_{1-20}$.

2. Procédé selon la revendication 1, **caractérisé en ce que** les nanotubes de carbone monoparois métalliques et semi-conducteurs présentent une longueur comprise entre 10 nm et 400 μm, bornes incluses, et un diamètre compris entre 0,2 et 6 nm, bornes incluses.

3. Procédé selon l'une des revendications 1 ou 2, **caractérisé en ce que** la concentration en nanotubes monoparois dans la dispersion aqueuse est comprise entre $1.10^{-5}$ et 10 g/l, bornes incluses.

4. Procédé selon l'une quelconque des revendications 1 à 3, **caractérisé en ce que** le tensioactif cationique est choisi parmi le bromure de dodécyltriméthylammonium, le bromure d'hexadécyltriméthylammonium, le bromure d'octadécyltriméthylammonium, le chlorure de dodécyltriméthylammonium, le chlorure d'hexadécylpyridinium, le poly(4-vinylpyridine-co-butylméthacrylate) ou le poly(4-vinylpyridine-co-styrène).

5. Procédé selon l'une quelconque des revendications 1 à 4, **caractérisé en ce que** le tensioactif cationique dans la dispersion aqueuse est présent en une quantité comprise entre 0,001 et 10%, bornes incluses, en masse par rapport à la masse totale de la dispersion.

6. Procédé selon l'une quelconque des revendications 1 à 5, **caractérisé en ce que** le pH est supérieur ou égal à 0 et inférieur à 5.

7. Procédé selon l'une quelconque des revendications 1 à 6, **caractérisé en ce que** le pH est maintenu constant par ajout d'une solution tampon acide choisie parmi $CH_3CO_2H/CH_3CO_2^-$, $H_3PO_4/H_2PO_4^-$ ou $C_6H_5CO_2H/C_6H_5CO_2^-$.

8. Procédé selon l'une quelconque des revendications 1 à 7, **caractérisé en ce que** la concentration en (Z)-diazoester ascorbique de formule (I) dans la dispersion, est de $1.10^{-9}$ à 1 mol/l.

9. Procédé selon l'une quelconque des revendications 1 à 8, **caractérisé en ce qu'**il est mis en oeuvre à une température comprise entre 0 et 50°C, bornes incluses.

10. Procédé selon l'une quelconque des revendications 1 à 9, **caractérisé en ce que** la durée de réaction entre ladite dispersion et le (Z)-diazoester ascorbique de formule (I) est comprise entre 5 minutes et 48 heures, bornes incluses.

11. Procédé selon l'une quelconque des revendications 1 à 10, **caractérisé en ce que** le (Z)-diazoester ascorbique de formule (I), est obtenu par réaction d'un sel de diazonium de formule (II) avec un composé d'acide ascorbique de formule (III).

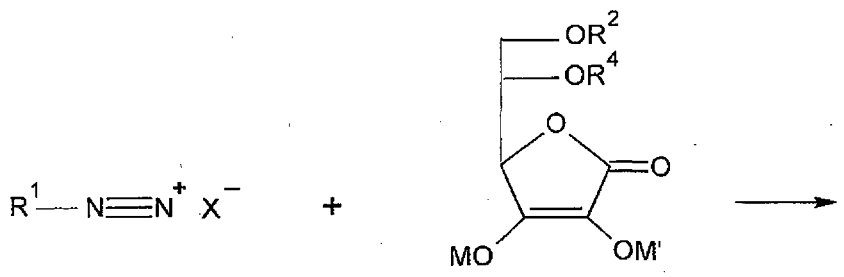

(II) (III) (I)

dans lesquelles

- $R^1$, $R^2$, et $R^4$ sont tels que définis à la revendication 1 ;
- M et M', identiques ou différents, sont choisis, indépendamment l'un de l'autre, parmi un atome d'hydrogène, un métal alcalin choisi parmi le sodium ou le potassium ;
- $X^-$ représente un atome d'halogène choisi parmi le fluor, le chlore, le brome ou l'iode ; ou un anion choisi parmi $BF_4^-$, $ClO_4^-$, ou $SO_3^-$.

**12.** Procédé selon l'une quelconque des revendications 1 à 11, **caractérisé en ce qu'**il est suivi d'une étape de séparation des nanotubes de carbone monoparois métalliques fonctionnalisés des nanotubes de carbone monoparois semi-conducteurs.

**13.** Procédé selon la revendication 12, **caractérisé en ce qu'**il comprend, en outre, une étape de traitement thermique desdits nanotubes.

**14.** Utilisation d'un mélange de nanotubes de carbone monoparois métalliques sélectivement fonctionnalisés et de nanotubes de carbone monoparois semi-conducteurs, obtenu par le procédé selon l'une quelconque des revendications 1 à 11, pour la réalisation de canaux de transistors, de matériaux accepteurs d'électrons, d'émetteurs ou absorbeurs non linéaires de photons infra-rouge, d'électrodes conductrices de courant, d'électrodes transparentes souples, de revêtements antistatiques, de détecteurs chimiques, et de cellules solaires.

**15.** Nanotubes de carbone monoparois métalliques fonctionnalisés obtenus par le procédé selon la revendication 12.

**16.** Utilisation de nanotubes de carbone monoparois métalliques fonctionnalisés selon la revendication 15, pour la réalisation d'électrodes conductrices de courant, d'électrodes transparentes souples, de revêtements antistatiques, de vias et interconnections en électronique, de câbles de conduction du courant, et de cellules solaires.

**17.** Procédé de séparation de nanotubes de carbone monoparois métalliques des nanotubes de carbone monoparois semi-conducteurs comprenant une étape de fonctionnalisation sélective des nanotubes de carbone monoparois métalliques selon le procédé décrit à l'une quelconque des revendications 1 à 11.

**Patentansprüche**

**1.** Verfahren zur selektiven Funktionalisierung von metallischen einwandigen Kohlenstoffnanoröhren, **dadurch gekennzeichnet, dass** bei pH < 5 umgesetzt wird:

- eine wässrige Dispersion, umfassend ein Gemisch aus metallischen einwandigen Kohlenstoffnanoröhren und halbleitenden einwandigen Kohlenstoffnanoröhren sowie ein kationisches Tensid, mit
- einem Ascorbin-(Z)-Diazoester der Formel (I):

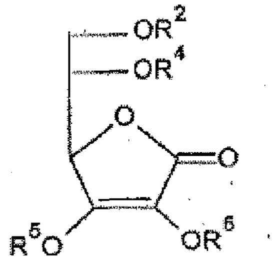

(I)

worin

- $R^5$ und $R^6$, die verschieden sind, unabhängig voneinander aus einem Wasserstoffatom oder einer $R^1$-N=N-Gruppe ausgewählt sind;
- $R^1$ für eine $C_{6-10}$-Arylgruppe oder eine Pyridylgruppe steht, wobei die Aryl- und Pyridylgruppen gegebenenfalls durch einen oder mehrere Substituenten substituiert sind, die aus einem aus Chlor oder Brom ausgewählten Halogenatom, $-NO_2$, $-CO_2H$, $-CO_2R^3$, $-R^3$ oder $-OR^3$ ausgewählt sind;
- $R^2$ und $R^4$, die identisch oder verschieden sind, unabhängig voneinander aus einem Wasserstoffatom, einer $C_{1-20}$-Alkylgruppe, -CO-Alkyl-($C_{1-20}$), $-R^3$, $-CO_2R^3$, $-CO-NHR^3$, $-CO-NR^3R^7$ ausgewählt sind;
- $R^3$ und $R^7$, die identisch oder verschieden sind, unabhängig voneinander aus einer $C_{1-20}$-Alkylgruppe oder einer $C_{1-20}$-Halogen-Alkylgruppe ausgewählt sind.

**2.** Verfahren nach Anspruch 1, **dadurch gekennzeichnet, dass** die metallischen und halbleitenden einwandigen Kohlenstoffnanoröhren eine Länge zwischen 10 nm und 400 $\mu$m, Grenzwerte eingeschlossen, und einen Durchmesser zwischen 0,2 und 6 nm, Grenzwerte eingeschlossen, aufweisen.

**3.** Verfahren nach einem der Ansprüche 1 und 2, **dadurch gekennzeichnet, dass** die Konzentration der einwandigen Nanoröhren in der wässrigen Dispersion zwischen $1 \cdot 10^{-5}$ und 10 g/l, Grenzwerte eingeschlossen, beträgt.

**4.** Verfahren nach einem der Ansprüche 1 bis 3, **dadurch gekennzeichnet, dass** das kationische Tensid aus Dodecyltrimethylammoniumbromid, Hexadecyltrimethylammoniumbromid, Octadecyltrimethylammoniumbromid, Dodecyltrimethylammoniumchlorid, Hexadecylpyridiniumchlorid, Poly(4-Vinylpyridin-co-butylmethacrylat) oder Poly(4-Vinylpyridin-co-styrol) ausgewählt ist.

**5.** Verfahren nach einem der Ansprüche 1 bis 4, **dadurch gekennzeichnet, dass** das kationische Tensid in der wässrigen Dispersion in einer Menge von 0,001 bis 10 Gew.-%, Grenzwerte eingeschlossen, bezogen auf das Gesamtgewicht der Dispersion vorliegt.

**6.** Verfahren nach einem der Ansprüche 1 bis 5, **dadurch gekennzeichnet, dass** der pH-Wert größer oder gleich 0 und kleiner 5 ist.

**7.** Verfahren nach einem der Ansprüche 1 bis 6, **dadurch gekennzeichnet, dass** der pH-Wert durch Zugabe einer sauren Pufferlösung, die ausgewählt ist aus $CH_3CO_2H/CH_3CO_2^-$, $H_3PO_4/H_2PO_4^-$ oder $C_6H_5CO_2H/C_6H_5CO_2^-$, konstant gehalten wird.

**8.** Verfahren nach einem der Ansprüche 1 bis 7, **dadurch gekennzeichnet, dass** die Konzentration des Ascorbin-(Z)-Diazoesters der Formel (I) in der Dispersion $1 \cdot 10^{-9}$ bis 1 mol/l beträgt.

**9.** Verfahren nach einem der Ansprüche 1 bis 8, **dadurch gekennzeichnet, dass** es bei einer Temperatur zwischen 0 und 50°C, Grenzwerte eingeschlossen, durchgeführt wird.

**10.** Verfahren nach einem der Ansprüche 1 bis 9, **dadurch gekennzeichnet, dass** die Dauer der Reaktion zwischen der Dispersion und dem Ascorbin-(Z)-Diazoester der Formel (I) zwischen 5 Minuten und 48 Stunden, Grenzwerte eingeschlossen, beträgt.

**11.** Verfahren nach einem der Ansprüche 1 bis 10, **dadurch gekennzeichnet, dass** das Ascorbin-(Z)-Diazoester der

Formel (I) durch Umsetzen eines Diazoniumsalzes der Formel (II) mit einer Ascorbinsäureverbindung der Formel (III) erhalten wird.

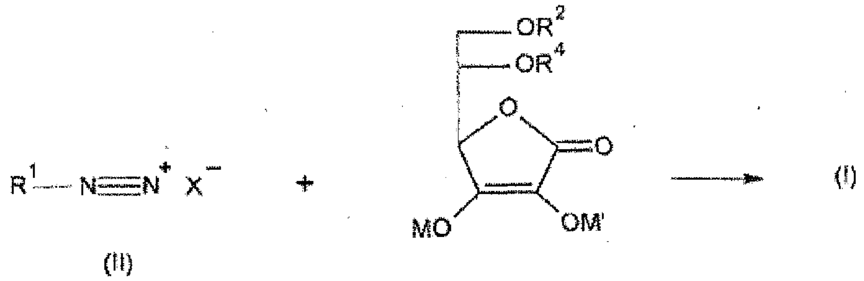

(II)

(III)

worin

- $R^1$, $R^2$ und $R^4$ wie in Anspruch 1 definiert sind;
- M und M', die identisch oder verschieden sind, unabhängig voneinander aus einem Wasserstoffatom, einem aus Natrium oder Kalium ausgewählten Alkalimetall ausgewählt sind;
- X- für ein Halogenatom steht, das aus Fluor, Chlor, Brom oder Jod ausgewählt ist, oder für ein Anion, das aus $BF_4^-$, $ClO_4^-$ oder $SO_3^-$ ausgewählt ist.

**12.** Verfahren nach einem der Ansprüche 1 bis 11, **dadurch gekennzeichnet, dass** es von einem Schritt der Trennung der funktionalisierten, metallischen einwandigen Kohlenstoffnanoröhren von den halbleitenden einwandigen Kohlenstoffnanoröhren gefolgt ist.

**13.** Verfahren nach Anspruch 12, **dadurch gekennzeichnet, dass** es zudem einen Schritt der Wärmebehandlung der Nanoröhren umfasst.

**14.** Verwendung eines Gemischs aus selektiv funktionalisierten, metallischen einwandigen Kohlenstoffnanoröhren und halbleitenden einwandigen Kohlenstoffnanoröhren, erhalten durch das Verfahren nach einem der Ansprüche 1 bis 11, zur Realisierung von Transistorkanälen, elektronenaufnehmenden Materialien, nicht-linearen Emittern oder Absorbern infraroter Photonen, stromleitenden Elektroden, flexiblen transparenten Elektroden, antistatischen Beschichtungen, chemischen Sensoren und Solarzellen.

**15.** Funktionalisierte, metallische einwandige Kohlenstoffnanoröhren, erhalten durch das Verfahren nach Anspruch 12.

**16.** Verwendung von funktionalisierten, metallischen einwandigen Kohlenstoffnanoröhren nach Anspruch 15 zur Realisierung von stromleitenden Elektroden, flexiblen transparenten Elektroden, antistatischen Beschichtungen, Durchkontaktierungen und Verbindungen in der Elektronik, stromführenden Kabeln und Solarzellen.

**17.** Verfahren zur Trennung von metallischen einwandigen Kohlenstoffnanoröhren von halbleitenden einwandigen Kohlenstoffnanoröhren, umfassend einen Schritt der selektiven Funktionalisierung der metallischen einwandigen Kohlenstoffnanoröhren gemäß dem in einem der Ansprüche 1 bis 11 beschriebenen Verfahren.

## Claims

**1.** Process for the selective functionalisation of metallic single-walled carbon nanotubes, **characterised in that**:

- an aqueous dispersion comprising a mixture of metallic single-walled carbon nanotubes and of semi-conducting single-walled carbon nanotubes and a cationic surfactant is reacted at pH < 5 with
- an ascorbic (Z)-diazo ester of formula (I):

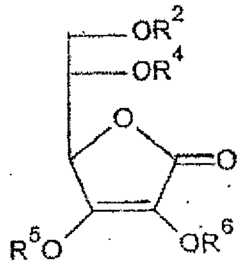

(I)

wherein

- $R^5$ and $R^6$, which are different, are chosen independently of one another from a hydrogen atom and a group $R^1$-N=N-;
- $R^1$ represents a $C_{6-10}$-aryl group or a pyridyl group, said aryl and pyridyl groups being optionally substituted by one or more substituents chosen from a halogen atom chosen from chlorine and bromine, $-NO_2$, $-CO_2H$, $-CO_2R^3$, $-R^3$ and $-OR^3$;
- $R^2$ and $R^4$, which may be identical or different, are chosen independently of one another from a hydrogen atom, a group $C_{1-20}$-alkyl, -CO-$C_{1-20}$-alkyl, $-R^3$, $-CO_2R^3$, $-CO-NHR^3$, $-CO-NR^3R^7$;
- $R^3$ and $R^7$, which may be identical or different, are chosen independently of one another from a $C_{1-20}$-alkyl group and a halo-$C_{1-20}$-alkyl group.

**2.** Process according to claim 1, **characterised in that** the metallic and semi-conducting single-walled carbon nanotubes have a length of from 10 nm to 400 $\mu$m inclusive and a diameter of from 0.2 to 6 nm inclusive.

**3.** Process according to either claim 1 or claim 2, **characterised in that** the concentration of single-walled nanotubes in the aqueous dispersion is from $1\times10^{-5}$ to 10 g/l inclusive.

**4.** Process according to any one of claims 1 to 3, **characterised in that** the cationic surfactant is chosen from dodecyltrimethylammonium bromide, hexadecyltrimethylammonium bromide, octadecyltrimethylammonium bromide, dodecyltrimethylammonium chloride, hexadecylpyridinium chloride, poly(4-vinylpyridine-co-butyl methacrylate) and poly(4-vinylpyridine-co-styrene).

**5.** Process according to any one of claims 1 to 4, **characterised in that** the cationic surfactant in the aqueous dispersion is present in an amount by mass of from 0.001 to 10% inclusive, based on the total mass of the dispersion.

**6.** Process according to any one of claims 1 to 5, **characterised in that** the pH is greater than or equal to 0 and less than 5.

**7.** Process according to any one of claims 1 to 6, **characterised in that** the pH is kept constant by addition of an acidic buffer solution chosen from $CH_3CO_2H/CH_3CO_2^-$, $H_3PO_4/H_2PO_4^-$ and $C_6H_5CO_2H/C_6H_5CO_2^-$.

**8.** Process according to any one of claims 1 to 7, **characterised in that** the concentration of ascorbic (Z)-diazo ester of formula (I) in the dispersion is from $1\times10^{-9}$ to 1 mol/l.

**9.** Process according to any one of claims 1 to 8, **characterised in that** it is carried out at a temperature of from 0 to 50°C inclusive.

**10.** Process according to any one of claims 1 to 9, **characterised in that** the duration of the reaction between said dispersion and the ascorbic (Z)-diazo ester of formula (I) is from 5 minutes to 48 hours inclusive.

**11.** Process according to any one of claims 1 to 10, **characterised in that** the ascorbic (Z)-diazo ester of formula (I) is obtained by reacting a diazonium salt of formula (II) with an ascorbic acid compound of formula (III)

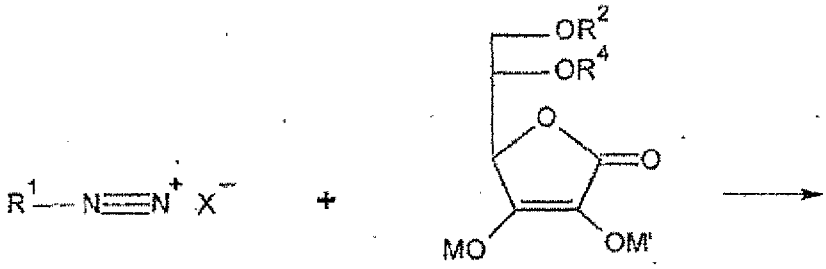

(II) (III) (I)

wherein

- $R^1$, $R^2$ and $R^4$ are as defined in claim 1;
- M and M', which may be identical or different, are chosen independently of one another from a hydrogen atom and an alkali metal chosen from sodium and potassium;
- $X^-$ represents a halogen atom chosen from fluorine, chlorine, bromine and iodine; or an anion chosen from $BF_4^-$, $ClO_4^-$ and $SO_3^-$.

**12.** Process according to any one of claims 1 to 11, **characterised in that** it is followed by a step of separating the functionalised metallic single-walled carbon nanotubes from the semi-conducting single-walled carbon nanotubes.

**13.** Process according to claim 12, **characterised in that** it further comprises a step of treating said nanotubes by heat.

**14.** Use of a mixture of selectively functionalised metallic single-walled carbon nanotubes and of semi-conducting single-walled carbon nanotubes, obtained by the process according to any one of claims 1 to 11, in the production of transistor channels, electron acceptor materials, non-linear infra-red photon emitters or absorbers, current conducting electrodes, flexible transparent electrodes, antistatic coatings, chemical detectors and solar cells.

**15.** Functionalised metallic single-walled carbon nanotubes obtained by the process according to claim 12.

**16.** Use of functionalised metallic single-walled carbon nanotubes according to claim 15 in the production of current conducting electrodes, flexible transparent electrodes, antistatic coatings, vias and interconnects in electronics, current conducting cables and solar cells.

**17.** Process for the separation of metallic single-walled carbon nanotubes from semi-conducting single-walled carbon nanotubes, comprising a step of selectively functionalising the metallic single-walled carbon nanotubes according to the process described in any one of claims 1 to 11.

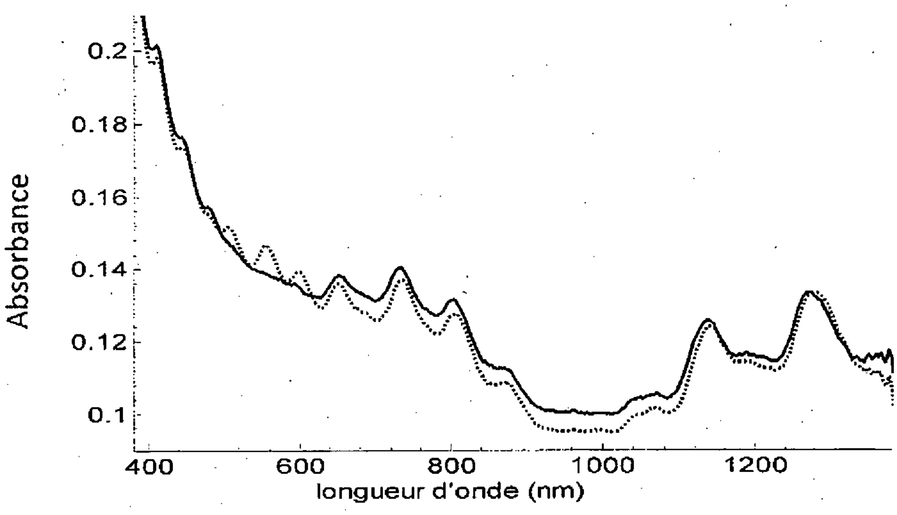


SWNTs HiPCo®

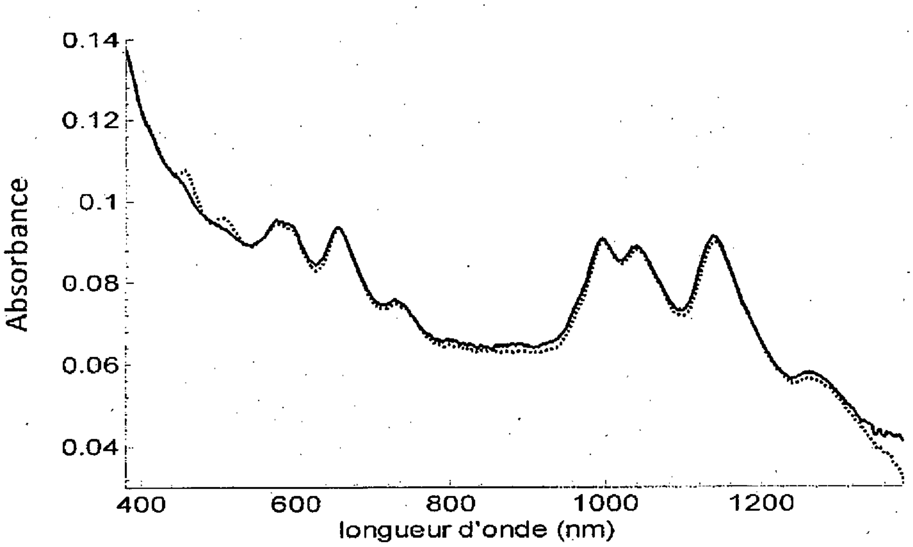


SWNTs CoMocat®

**Figure 1**

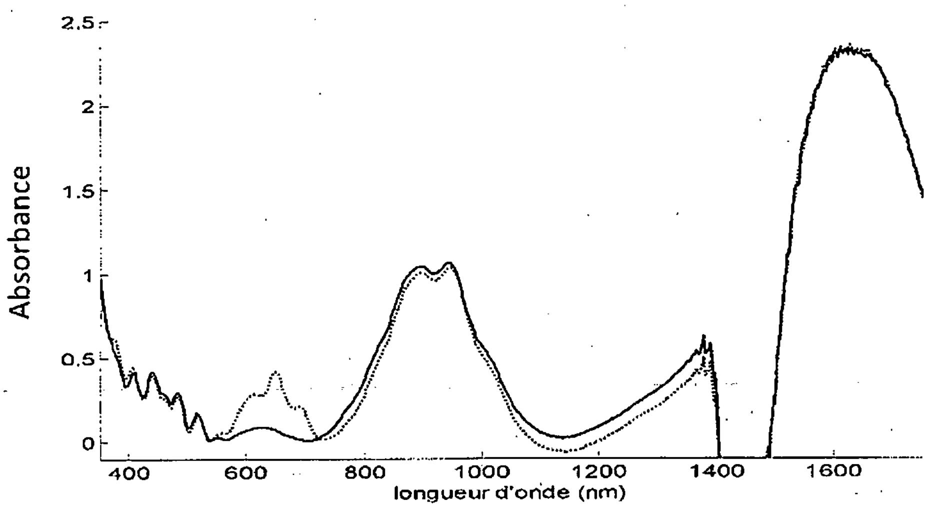


SWNTs Laser

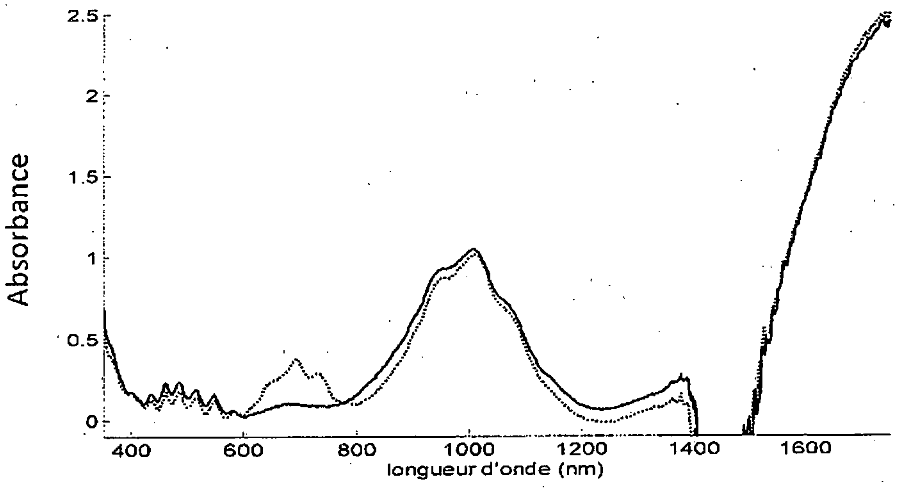


SWNTs NanoCarb®

**Figure 2**

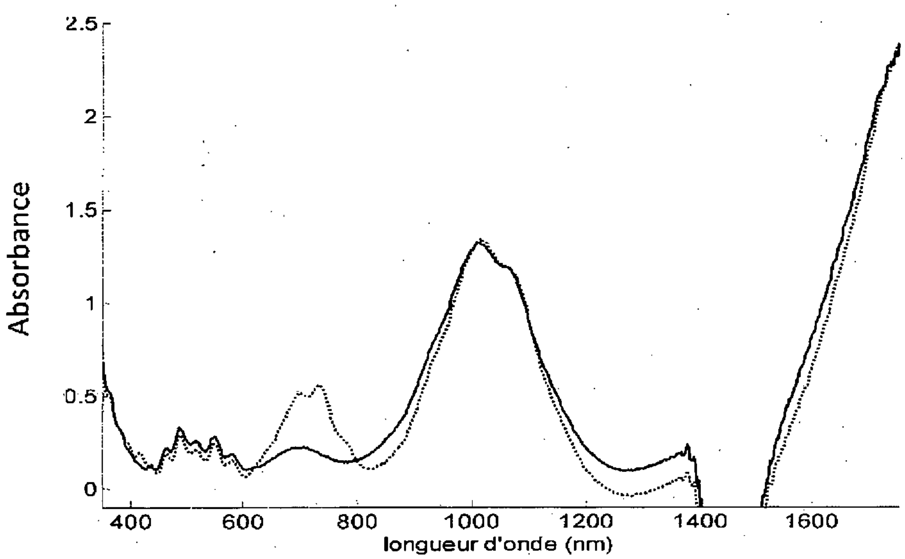


SWNTs Carbon Solutions

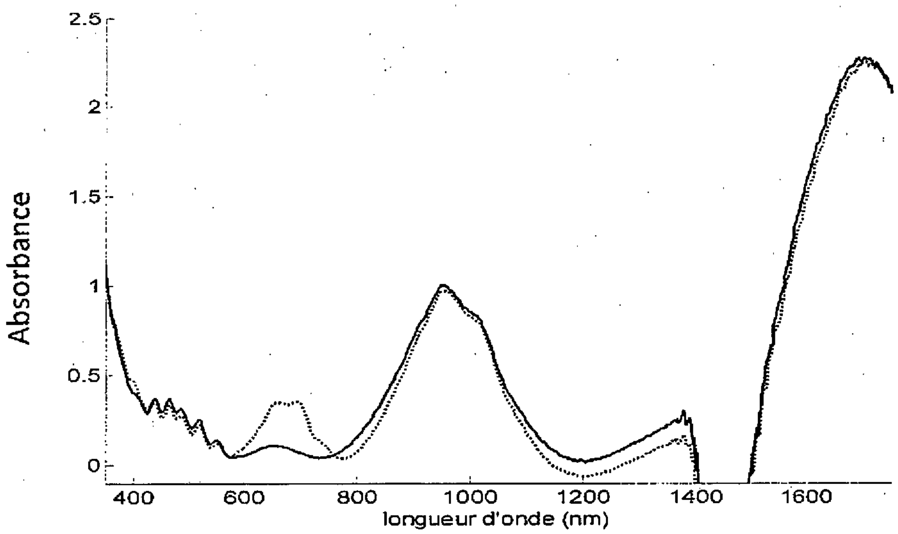


SWNTs NanoLedge

**Figure 3**

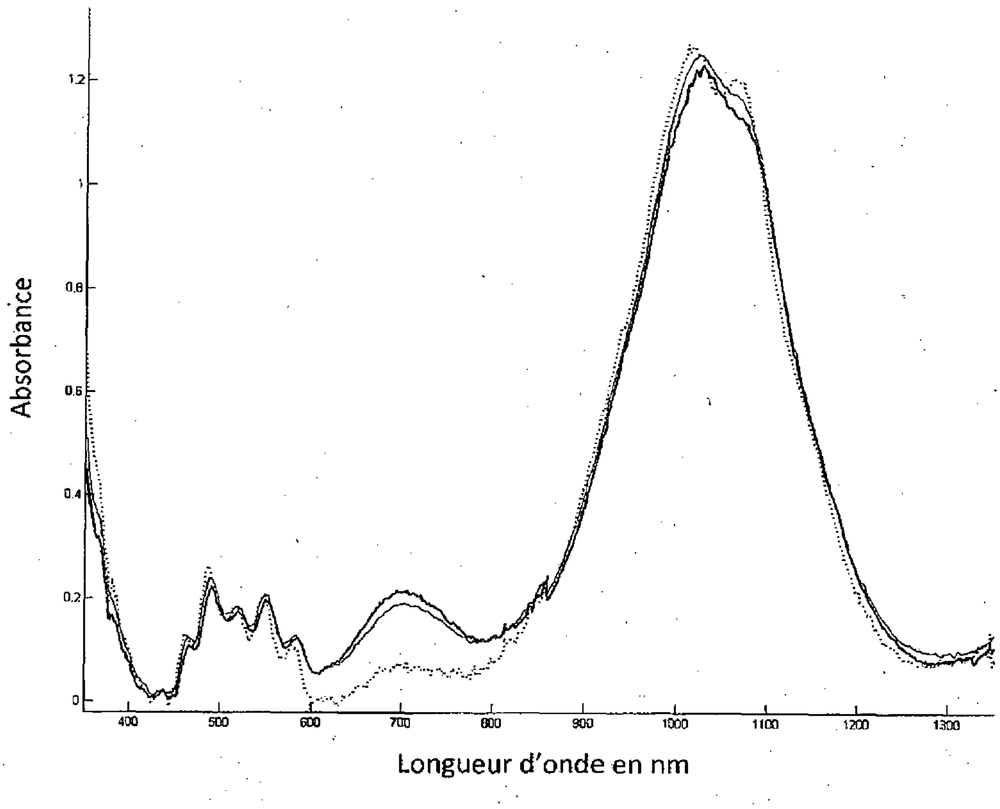
Absorbance
Longueur d'onde en nm
1.2
1
0.8
0.6
0.4
0.2
0
400
500
600
700
800
900
1000
1100
1200
1300

**Figure 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2010089395 A
- JP 2007031238 A


### Littérature non-brevet citée dans la description


- **STRANO MS. et al.** *Science,* 2003, vol. 301, 1519-1522
- **DYKE CA. et al.** *Journal of the American Chemical Society,* 2005, vol. 127, 4497-4509
- **DOYLE CD et al.** *Journal of the American Chemical Society,* 2008, vol. 130, 6795-6800
- **NAIR N. et al.** *Journal of the American Chemical Society,* 2007, vol. 129, 3946-3954
- **WANG CJ. et al.** *Journal of the American Chemical Society,* 2005, vol. 127, 11460-11468
- **GHOSH S. et al.** *Nano Research,* 2009, vol. 2, 183-191
- **LEE CW. et al.** *Advanced Materials,* 2010, vol. 22, 1278
- **ZHANG L et al.** *Journal of the American Chemical Society,* 2008, vol. 130, 2686-2691
- **SCHMIDT G. et al.** *Chemistry European Journal,* 2011, vol. 17, 1415-1418
- **CABANA J. et al.** *Journal of the American Chemical Society,* 2007, vol. 129, 2244
- **TANAKA T et al.** *Nano Letters,* 2009, vol. 9, 1497-1500
- **MOSHAMMER K. et al.** *Nano Research,* 2009, 599-606
- **ARNOLD MS. et al.** *Nature Nanotechnology,* 2006, vol. 1, 60-65
- **O. JOST et al.** *Applied Physics Letters,* 1999, vol. 75, 2217
- **ZOLLINGER H.** *VCH,* 1994, 108-115
- **DOYLE MP. et al.** *Journal of Organic Chemistry,* 1989, vol. 54, 3785-3789
- **G. SCHMIDT et al.** *Chemistry - a European Journal,* 2011, vol. 17, 1415
- **DYKE C.A et al.** *Synlett,* 2004, vol. 1, 155-160